# EUROPEAN PATENT SPECIFICATION

(11) **EP 3 489 958 B1**
(45) Date of publication and mention of the grant of the patent: **16.03.2022**
(21) Application number: 17203294.8
(22) Date of filing: 23.11.2017
(51) Int. Cl.: G16H 10/60, G16H 50/70

(54) **HEALTHCARE NETWORK**
GESUNDHEITSVERSORGUNGSNETZWERK
RÉSEAU DE SOINS DE SANTÉ

(43) Date of publication of application: 29.05.2019
(73) Proprietor: Siemens Healthcare GmbH, 91052 Erlangen (DE)
(72) Inventor: Hartung, Ulrich, 91094 Langensendelbach (DE); Spitzner, Christian, 91301 Forchheim (DE); Markuszewska, Iwona, 91301 Forchheim (DE)

(56) References cited:
- US-A1- 2010 332 256
- US-A1- 2011 218 821

## Description

Examples described herein relate particularly, but not exclusively, to systems, methods and computer programs operable to provide healthcare data to a user device in a healthcare network.

A healthcare network enables accumulation and/or provisioning of healthcare, clinical and/or medical data. The healthcare network comprises a plurality of systems including devices and software applications. The devices may include medical devices (e.g. an ultrasound system), user devices for use by medical professionals and/or patients, servers and medical data sources. Software applications operate on, or in conjunction with or are accessible via, the devices, and may include communication applications that facilitate communication between devices on the healthcare network, medical applications configured to process healthcare data, applications configured to manage patient information, applications for managing knowledge in a healthcare environment, and applications configured to manage medical records.

Typically, a healthcare network comprises several disparate proprietary, off-the-shelf, structured and/or unstructured data sources, which are rarely integrated so as to operate homogeneously. A healthcare professional, when analysing healthcare data associated with a patient, may need healthcare data from different data sources at different stages of the analysis. In addition, healthcare professionals from different disciplines may need different healthcare data for their analysis. For example, different members of a tumour board, which is a multidisciplinary group of healthcare professionals that reviews ongoing cancer cases, may need access to different healthcare data. However, healthcare professionals have to manually retrieve healthcare data from various data sources to get all the relevant information required. An example of providing healthcare data can be seen in US 2010/332256 A1. In said document every single new data added to the networked system is indexed. Said document does not consider the sole indexing of requested data.

### Summary

In a first aspect, there is provided a system operable to provide healthcare data to a user device in a healthcare network, the healthcare network comprising a plurality of data sources comprising healthcare data, the system comprising at least one processor and at least one memory including computer program code, the at least one memory and the computer program code configured to, with the at least one processor, cause the system at least to perform the steps of:
maintaining, in a database, a first plurality of associations between a first plurality of the data sources and healthcare data stored therein;
receiving, from the user device, a request for first healthcare data, wherein the requested first healthcare data is stored in a second of the data sources, the second data source being different to the first plurality of data sources;
determining whether the requested first healthcare data can be retrieved based on the first plurality of associations;
selectively, based on an outcome of the determining, causing a second association between the second data source and the requested first healthcare data to be detected and stored in the database;
retrieving the first requested healthcare data from the second data source; and
transmitting the first retrieved healthcare data for receipt by the user device.

Therefore, the system advantageously integrates disparate data sources by maintaining associations between various data sources and healthcare data stored therein. If the requested healthcare data cannot be retrieved based on the maintained associations (i.e. the first plurality of associations), then the system may cause the second association to be detected, thereby selectively causing unknown data source(s) to be identified and associations in respect thereof to be stored. Subsequent requests for healthcare data from the second data source may be processed by the system using the second association, which is now known thereto, by retrieving and transmitting the requested healthcare data for receipt by the user device.

In the event that an association corresponding to requested healthcare data can be identified on the basis of the first plurality of associations, the system retrieves and transmits the requested healthcare data for receipt by the user device. Therefore, the system causes the second association to be selectively identified in dependence on whether the requested healthcare data can be retrieved based on the first plurality of associations, thereby increasing average response times for responding to requests for healthcare data.

The system may use an application detect which of the data sources comprises the requested healthcare data, thereby detecting the second association. The application may have crawling functionality. Therefore, embodiments enable a healthcare data provisioning framework in which the system operates alongside an application configured to detect associations that are unknown to the system.

The system may additionally remove an association from the first plurality of associations, thereby ceasing access to the corresponding healthcare data via the healthcare network. The system may, in response to receiving data indicative of a change between the second data source and healthcare data stored therein, update the second association, thereby continuing to facilitate access to the healthcare data stored in the second data source via the healthcare network. Therefore, embodiments facilitate access to up-to-date healthcare data.

The system may create one or more virtual maps based on the known associations, thereby creating one or more patient models. The or each patient model links related healthcare data. The system may use the patient model(s) to acquire relevant healthcare data in response to a request therefor.

The system may maintain a state parameter indicative of a current stage of analysis, and use it to cause at least part of the retrieved healthcare data to be displayed on a user terminal. The state parameter may, for example, be indicative of a clinical stage, such diagnosis, treatment etc.

The system may monitor prior actions of a user when analysing healthcare data associated with at least some of the different stages and may control display of at least a part of the acquired healthcare data on the user device based on a combination of the state parameter and the monitoring. Therefore, embodiments reduce cognitive burden by pre-processing acquired healthcare data based on a user's style of working. The system may, in response to a user input, cause the user device to acquire further healthcare data based on a combination of the input and the state parameter and using the patient model, and may cause the user device to present at least a part of the acquired further healthcare data. Therefore, embodiments are responsive to inputs from users at various stages, and use the inputs to automatically acquire and display further healthcare data. The system may, additionally or alternatively, process the retrieved healthcare data based on a predetermined parameter, and thereafter cause at least a part of the processed healthcare data to be presented. The predetermined parameter may, for example, comprise data indicative of a relevant frame of reference, such as a patient, for use in pre-processing of healthcare data prior to presentation on a user device.

The system may, additionally or alternatively, maintain a context parameter indicative of a clinical step associated with analysis of the received data and is a step in a clinical stage or process. For example, the context parameter may be indicative of a step of premedication in a clinical process of bronchoscopy.

The system may control display of at least a part of the filtered healthcare data on the user device based on a combination of the state parameter and the context parameter, thereby providing relevant healthcare date for a current step of assessment.

In a second aspect, there is provided a method of providing healthcare data to a user device in a healthcare network, the healthcare network comprising a plurality of data sources comprising healthcare data, the method comprising:
maintaining, in a database, a first plurality of associations between a first plurality of the data sources and healthcare data stored therein;
receiving, from the user device, a request for first healthcare data, wherein the requested first healthcare data is stored in a second of the data sources, the second data source being different to the first plurality of data sources;
determining whether the requested first healthcare data can be retrieved based on the first plurality of associations;
selectively, based on an outcome of the determining, causing a second association between the second data source and the requested first healthcare data to be detected and stored in the database;
retrieving the requested healthcare data from the second data source; and
transmitting the retrieved first healthcare data for receipt by the user device.

In a third aspect, there is provided a computer program comprising a set of instructions, which, when executed by a computerised device, cause the computerised device to perform a method of providing healthcare data to a user device in a healthcare network, the healthcare network comprising a plurality of data sources comprising healthcare data, the method comprising, at the computerised device:
maintaining, in a database, a first plurality of associations between a first plurality of the data sources and healthcare data stored therein;
receiving, from the user device, a request for first healthcare data, wherein the requested first healthcare data is stored in a second of the data sources, the second data source being different to the first plurality of data sources;
determining whether the requested first healthcare data can be retrieved based on the first plurality of associations;
selectively, based on an outcome of the determining, causing a second association between the second data source and the requested first healthcare data to be detected and stored in the database;
retrieving the first requested healthcare data from the second data source; and
transmitting the retrieved first healthcare data for receipt by the user device.

In a fourth aspect not covered by the claimed invention, there is provided a system operable to provide healthcare data to a user device in a healthcare network, the healthcare network comprising a plurality of data sources comprising healthcare data, the system comprising at least one processor and at least one memory including computer program code, the at least one memory and the computer program code configured to, with the at least one processor, cause the system at least to perform the steps of:
maintaining, in a database, a plurality of patient models, each patient model comprising a virtual map associating healthcare data related to at least one patient and a first plurality of the data sources;
receiving, from the user device, a request for healthcare data;
identifying at least one patient model in dependence on the requested healthcare data;
retrieving the requested healthcare data using the identified at least one patient model; and
transmitting the retrieved healthcare data for receipt by the user device.

Therefore, the system advantageously integrates disparate data sources by maintaining patient models comprising a virtual map associating healthcare data related to patient(s) and the first plurality of associations, and using the relevant patient model(s) for retrieving the requested healthcare data.

In the event that an association corresponding to requested healthcare data can be identified using the patient model(s), the system retrieves and transmits the requested healthcare data for receipt by the user device. If the requested healthcare data cannot be retrieved using the patient model(s), then the system causes a second association between a second data source and the requested healthcare data stored therein to be detected, wherein the requested healthcare data is stored in the second data source. Therefore, the system causes the second association to be selectively detected in dependence on whether the requested healthcare data can be retrieved based on the first plurality of associations, thereby increasing average response times for responding to requests for healthcare data.

The system may use an application to detect which of the data sources comprises the requested healthcare data, thereby detecting the second association. The application may have crawling functionality. Therefore, embodiments enable a healthcare data provisioning framework in which the system operates alongside an application configured to detect associations that are unknown to the system.

The system may additionally remove an association from the first plurality of associations, thereby ceasing access to the corresponding healthcare data via the healthcare network. In this case, the system may update the relevant of the patient model(s) subsequent to removal of an association.

The system may, in response to receiving data indicative of a change between the second data source and healthcare data stored therein, update the second association and the relevant patient model(s), thereby continuing to facilitate access to the healthcare data stored in the second data source via the healthcare network. Therefore, embodiments facilitate access to up-to-date healthcare data.

The system may maintain a state parameter indicative of a current stage of analysis, and use it to cause at least part of the retrieved healthcare data to be displayed on a user terminal. The state parameter may, for example, be indicative of a clinical stage, such diagnosis, treatment etc.

The system may monitor prior actions of a user when analysing healthcare data associated with at least some of the different stages and may control display of at least a part of the acquired healthcare data on the user device based on a combination of the state parameter and the monitoring. Therefore, embodiments reduce cognitive burden by pre-processing acquired healthcare data based on a user's style of working. The system may, in response to a user input, cause the user device to acquire further healthcare data based on a combination of the input and the state parameter and using the relevant patient model(s), and may cause the user device to present at least a part of the acquired further healthcare data. Therefore, embodiments are responsive to inputs from users at various stages, and use the inputs to automatically acquire and display further healthcare data. The system may, additionally or alternatively, process the retrieved healthcare data based on a predetermined parameter, and thereafter cause at least a part of the processed healthcare data to be presented. The predetermined parameter may, for example, comprise data indicative of a relevant frame of reference, such as a patient, for use in pre-processing of healthcare data prior to presentation on a user device.

The system may, additionally or alternatively, maintain a context parameter indicative of a clinical step associated with analysis of the received data and is a step in a clinical stage or process. For example, the context parameter may be indicative of a step of premedication in a clinical process of bronchoscopy.

The system may control display of at least a part of the filtered healthcare data on the user device based on a combination of the state parameter and the context parameter, thereby providing relevant healthcare date for a current step of assessment.

In a fifth aspect not covered by the claimed invention, there is provided a method of providing healthcare data to a user device in a healthcare network, the healthcare network comprising a plurality of data sources comprising healthcare data, the method comprising:
maintaining, in a database, a plurality of patient models, each patient model comprising a virtual map associating healthcare data related to at least one patient and a first plurality of the data sources;
receiving, from the user device, a request for healthcare data;
identifying at least one patient model in dependence on the requested healthcare data;
retrieving the requested healthcare data using the identified at least one patient model; and
transmitting the retrieved healthcare data for receipt by the user device.

In a sixth aspect not covered by the claimed invention, there is provided a computer program comprising a set of instructions, which, when executed by a computerised device, cause the computerised device to perform a method of providing healthcare data to a user device in a healthcare network, the healthcare network comprising a plurality of data sources comprising healthcare data, the method comprising, at the computerised device:
maintaining, in a database, a plurality of patient models, each patient model comprising a virtual map associating healthcare data related to at least one patient and a first plurality of the data sources;
receiving, from the user device, a request for healthcare data;
identifying at least one patient model in dependence on the requested healthcare data;
retrieving the requested healthcare data using the identified at least one patient model; and
transmitting the retrieved healthcare data for receipt by the user device.

### Brief Description of the Drawings

Figure 1 shows a schematic block diagram of an example of a healthcare network in accordance with examples;
Figure 2 shows a flow diagram depicting an example of processing data in accordance with examples;
Figure 3 shows a schematic block diagram of an example of a healthcare data provisioning framework in accordance with examples;
Figure 4 shows a schematic block diagram of an example of a healthcare data provisioning framework in accordance with examples;
Figure 5 shows an example patient model in accordance with examples;
Figure 6 shows a flow diagram depicting an example of processing data in accordance with examples;
Figure 7 shows a schematic block diagram of an example of an interface on a user device in accordance with examples; and
Figures 7a-7e present examples of the interface shown with reference to Figure 7.

### Detailed Description

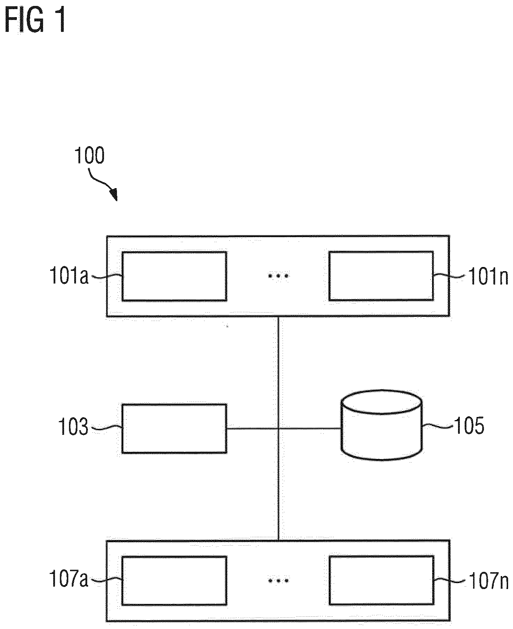

Referring to Figure 1, there is shown schematically an example of a healthcare network 100. The healthcare network 100 comprises a number of systems. The healthcare network 100 may correspond to systems in one healthcare institution or multiple healthcare institutions. The term "system" is used herein to denote an entity (or entities) in the healthcare network 100. A system may be embodied in the form of apparatus, hardware, software, a function, a virtualized resource, etc., or any combination of same. A healthcare network can comprise at least some different and/or additional components to those shown in Figure 1.

The healthcare network 100 comprises one or more user devices 101a-n configured for use by a healthcare professional or a patient. The user devices 101a-n may comprise a smart watch, a smart telephone, a tablet computer and/or a personal computer. The user devices 101a-n may comprise an application for presenting healthcare data. At least some of the user devices 101a-n may be communicatively coupled to each other via, for example, a local area network, metropolitan area network or a wide area network, and may have access to systems external to the healthcare network 100 via a Candidate Access Network (CAN) (not shown). The user devices 101a-n may comprise one or more processors (not shown) for performing various data processing operations according to examples and/or one or more memories (not shown) for storing various data according to examples.

The CAN may, for example, be a wireless access network or a wired access network. Examples of wireless access networks include, but are not limited to, Wireless Local Area Networks (WLANs) and mobile radio access networks. An example of a mobile radio access network is an Evolved Universal Terrestrial Radio Access Network (E-UTRAN). An example of a wired access network is an Asymmetric Digital Subscriber Line (ADSL) .

The healthcare network 100 may additionally comprise or facilitate access to one or more servers (not shown) configured to provision applications and data to the user devices 101a-n and other systems, a plurality of data sources 107a-n configured to provision healthcare data, a statistical decision support system, a clinical decision support system, a population health management system and/or one or more medical devices (for example, an ultrasound system).

The data sources 107a-n may be structured or unstructured, and may comprise a mix of disparate proprietary and off-the-shelf data sources that are located internally or externally to the healthcare network 100. The data sources 107a-n may, for example, include an Electronic Medical Records (EMR), a Picture Archiving Communication Systems (PACS), an Emergency Care Summary (ECS), a Key Information Summary (KIS), an Electronic Healthcare Record (EHR), an Electronic Patient Record (EPR) data source; and/or a Personal Health Record (PHR) data source. At least some of the data sources 107a-n may be configured to communicate via a corresponding communications interface providing a communications channel and/or an application programming interface for enabling communication therewith. The health industry standards do not mandate all operational aspects required for fully integrating data sources used in healthcare networks. In addition, there is very little guidance in the standards regarding integration of data sources with other systems in a healthcare network, specifically ones incorporating new technologies and/or involving multiple vendors. For example, some of the data sources 107a-n may conform to standards relevant for the medical industry, such as Health Level Seven (HL7) Fast Healthcare Interoperability Resources (FHIR) standard and/or Digital Imaging and Communications in Medicine (DICOM) standard, and/or conform to standards relevant for the digital communications industry, such as Representational State Transfer (REST) and/or Internet Protocol (IP).

At least some of the user devices 101a-n have access to a system 103 operable to provide healthcare data (also referred to as healthcare information). The healthcare data comprises clinical data (also referred to as clinical information) and/or medical data (also referred to as medical information). Clinical data comprises data collected during the course of ongoing patient care and/or as part of a formal clinical trial program, such as drug testing program. Clinical data may comprise: electronic health records of one or more patients, patient / disease registries indicative of data relating to chronic conditions such as Alzheimer's, clinical trials data, data for use in, for example, clinical or statistical decision support systems and/or anonymised patient medical records for, for example, use by a population health management system. Medical data comprises administrative, regulatory and R&D (research and development) data, guidelines and/or legal regulations.

The system 103 may be implemented on a single computer or a network of computers. In examples, the system 103 may be a central or location-specific distributed system. The system 103 may be located internally or externally to the healthcare network 100, and be operable to provide healthcare data to user devices or systems in one or a plurality of healthcare networks. The system 103 comprises one or more processors (not shown) for performing various data processing operations according to examples. The system 103 comprises or otherwise has access to a database 105 for storing various data according to examples. The database 105 may comprise one or more memories (not shown). The memory may be volatile so that data stored therein may need to be re-learnt upon failure/re-boot.

The database 105 maintains a first plurality of associations (also referred to as known associations) between a first plurality of the data sources 107a-n (also referred to as known data sources) and healthcare data stored therein. The mapping between the known data sources 107a-n and at least some of the healthcare data stored therein is recognised by the system 103, and can therefore be used for providing healthcare data to one or more of the user devices 101a-n and/or other systems, such as clinical or statistical decision support systems. The first plurality of associations may, for example, be known to the system 103 on the basis of one or more configuration parameters manually or automatically provided to the system 103 as part of the initial setup or otherwise. The system 103 may, additionally or alternatively, learn about associations between data sources and healthcare data stored therein using an application as will be described further below.

The known associations may be used by the system 103 to generate individual, collective, statistical, clinical or population patient models for use by healthcare professionals. The or each patient model is a virtual map of data sources recognised by the system 103 on the basis of the known associations, and is indicative of healthcare data relevant thereto. For example, an individual patient model comprises a virtual map associating healthcare data related to a patient based on the known associations. Collective patient models may correspond to a group of linked patients, such as patients linked by a blood relationship, and in this case the patient model comprises a virtual map associating healthcare data related to the group of patients based on the known associations. Statistical patient models may correspond to a group of patients sharing a clinical relationship, such as medical condition, for use in statistical decision support systems. Clinical patient models may correspond to a group of patients sharing a clinical relationship, such as medical condition, for use in clinical decision support systems. Population patient models may correspond to a group of related or unrelated patients for use in population health management systems, which aggregate anonymised patient data across one or more healthcare networks for use in monitoring and/or improving healthcare services. Data indicative of the or each patient model may be maintained in a system internal or external to the healthcare network, and may be used for retrieving healthcare data for provisioning to user devices in one or more healthcare networks.

In examples, in response to receiving a request for healthcare data from the user device 101, the system 103 may identify at least one of the patient models in dependence on the requested healthcare data. For example, if the request relates to healthcare data related to a patient, then the system 103 may identify an individual patient model associated with the patient. The system 103 may retrieve the requested healthcare data using the identified patient model(s). The system 103 may identify the relevant of the known data sources using the known associations, and retrieve relevant healthcare data therefrom. The system 103 may transmit the retrieved healthcare data for receipt by the user device 101. Therefore, enabling retrieval of healthcare data based on patient models generated based on known associations.

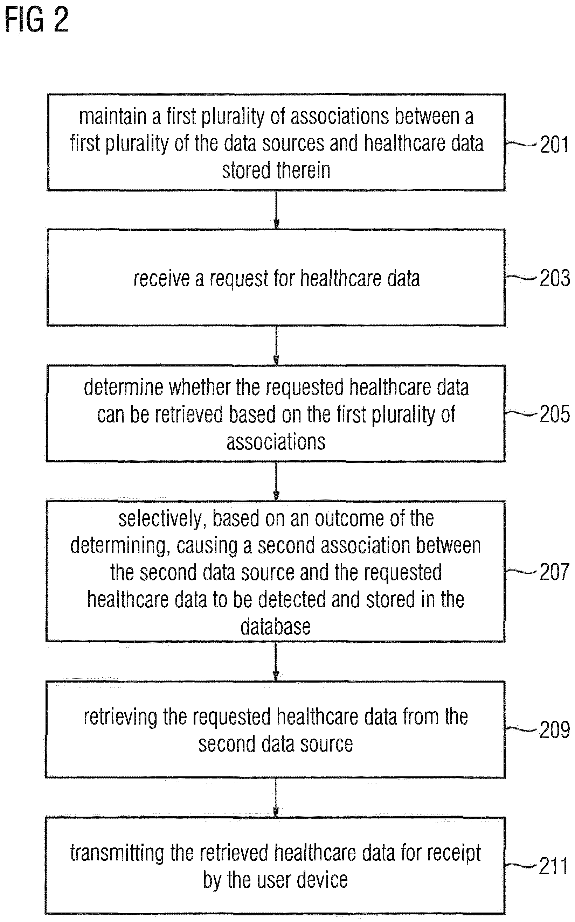

In the example set out with reference to Figure 2, the system 103 provides healthcare data to the user device 101 or a support system (not shown), including, but not limited to, a population health management system, a clinical decision support system and/or a statistical decision support system. In this example, the system 103 maintains the known associations in the database 107 (block 201).

The system 103 may receive a request from the user device 101 or the support system (as appropriate) for healthcare data (block 203). The request may, for example, include data identifying a patient, a healthcare professional, a medical condition and/or another type of healthcare data for use by the system 103 in identifying relevant healthcare data. The request may, for example, be received by the system 103 in response to an event, including, but not limited to, a healthcare professional interacting with an application on the user device 101 or the support system (as appropriate).

In examples, the system 103 may be able to provide the requested healthcare data from the known data sources on the basis of the known associations. However, in examples, the requested healthcare data may be stored in a second of the data sources 107, wherein the second data source is different to the known data sources in the sense that it is either not known to the system 103 or mapping between the second data source and the requested healthcare data is not known to the system 103. In examples, the second data source is logically distinct to the first plurality of sources.

The system 103 may determine whether the requested healthcare data can be retrieved based on the known associations (block 205). The system 103 may, for example, identify relevant patient model(s) and use the identified patient models to determine whether the requested healthcare data can be retrieved. In the event that relevant patient model cannot be identified, the system 103 may cause a relevant patient model to be generated. In the event that the requested healthcare data can be retrieved on the basis of the known associations, the system 103 may retrieve the requested healthcare data and transmit it to the user device 101 or the support system (as appropriate).

In this example, the system 103 is unable to retrieve the requested healthcare data on the basis of the known associations. The system 103, based on an outcome of the determining step at block 205, causes a second association between the second data source and the requested healthcare data to be detected and stored in the database 107 (block 207), thereby causing the second association to be one of the known associations. As will be described in further detail below, association in relation to unknown or as yet undetected data sources may be detected using an application having, for example, crawling functionality.

In at least some examples, responsive to storing the detected association in respect of the second data source and the healthcare data stored therein, the system 103 may update one or more of the relevant patient models to include healthcare data contained in the newly detected second data source, thereby causing the patient model(s) to be automatically updated and be comprehensive. In examples, the second data source may be a newly added data source in the healthcare network 100.

The system 103 may retrieve the requested healthcare data from the second data source (block 209), and transmit it for receipt by the user device 101 or the support system (as appropriate) (block 211). In examples, the retrieved healthcare data may be anonymised for confidentiality before transmission to the user device 101 or the support system (as appropriate).

Therefore, examples flexibly integrate disparate data sources accessible via the healthcare network 100. The integration is based on knowledge regarding associations between data sources and healthcare data stored therein. Using this knowledge, the system 103 generates and maintains a virtual map of data sources for use in retrieval of healthcare data. The map is updated as unknown or undetected data sources are detected or in response to identifying additional healthcare data stored in a known data source. Thus, examples enable aggregation of data from disparate data sources in a healthcare network without having to store healthcare data in a centralised repository.

In at least some examples, the system 103 may cause the second association to be detected if an association corresponding to the healthcare data requested at block 203 is not determined on the basis of the known associations at block 205. In this case, the system 103 determines that the requested healthcare data cannot be retrieved based on the known associations, and selectively causes the second association to be detected based on the outcome of the determining at block 205.

If an association corresponding to the requested healthcare data is determined on the basis of the known associations at block 205, the system 103 may retrieve the healthcare data requested at block 203 on the basis of the relevant one of the known associations and transmit it to the user device 101 or the support system (as appropriate). Therefore, the system 103 selectively causes an association to be identified if the requested healthcare data cannot be retrieved based on the known associations.

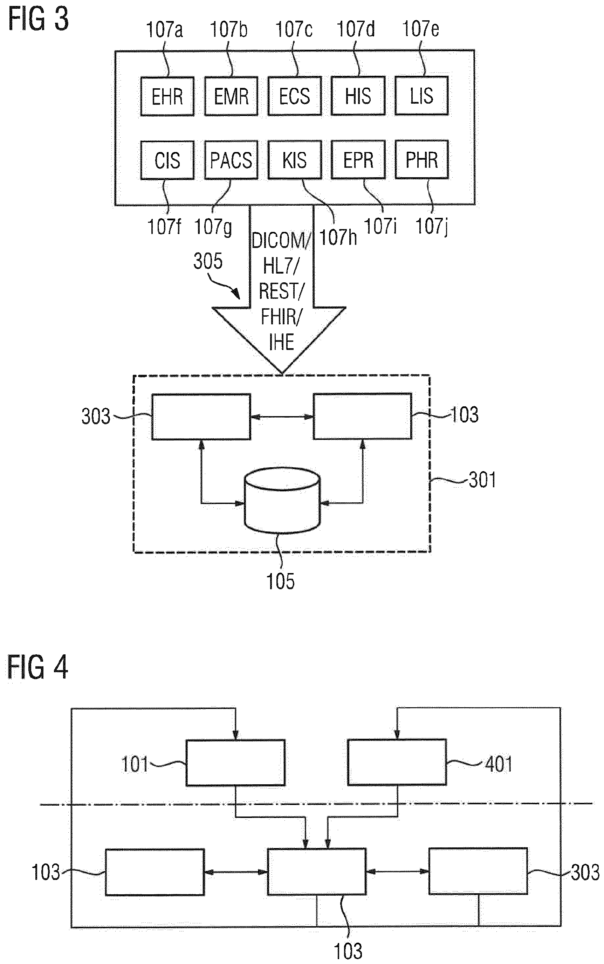

In the example set forth in Figure 3, the system 103 uses an application 303 to detect which of the data sources 107 comprises the healthcare data requested at block 203, thereby causing detection of the second association. The application 303 may conceptually be a part of a healthcare data provisioning framework 301 configured to provision healthcare data to user devices and/or systems in one or more healthcare networks. The framework 301 additionally comprises the system 103 and the database 105.

In this example, the healthcare network 100 has access to: an EHR 107a, an EMR 107b, an ECS 107c, a HIS 107d, a LIS 107e, a CIS 107f, a PACS 107g, a KIS 107h, an EPR 107i and a PHR 107j. Communication between the data sources 107a-j may be via a standardised or a proprietary communications interface or an application programming interface 305. The interface may, for example, conform to one or more of standards: DICOM; HL7; REST; FHIR; IP; and/or IHE. In effect, the framework 301 communicates with the various data sources 107a-j using supported interfaces, but hides implementation specific details, including supported communication interfaces, from the user devices 101 and the support systems requesting healthcare data therefrom.

In examples, the application 303 may be a crawler (also referred to as bot or spider) comprising crawling functionality. Crawling process comprises detecting unknown data sources and/or new or updated content in known data sources. The application 303 may, additionally or alternatively, index healthcare data stored in newly detected data sources, thereby identifying relationship between a newly detected data source and healthcare data stored therein. The application may, additionally or alternatively, rank data sources based on, for example, reliability and healthcare data stored therein, and use the ranking data for filtering healthcare data when responding to requests for healthcare data. The application may, additionally or alternatively, use artificial intelligence and/or machine learning techniques to detect unknown data sources.

The application 303 may, additionally or alternatively, detect unknown data sources on the basis of a message queue, via which at least some of the data sources 107a-j announce or broadcast their presence. The application 303 may, additionally or alternatively, be configured to detect periodic beacon signals transmitted by one or more of the data sources 107a-j, in order to detect unknown data sources. For example, the application 303 may listen to an HL7 version 2 message queue and use the messages transmitted thereon them for notifying the system 303 of any updates in relation to known or unknown data sources and/or detecting unknown data sources.

The application 303 may, additionally or alternatively, be configured to conduct a capability negotiation exchange with at least some of the newly detected data sources 107a-j (as appropriate). The capability negotiation may, for example, involve detecting supported communication protocols, healthcare data stored and schema used for storing healthcare data, and may store it in a profile associated with the newly detected data source, thereby facilitating seamless access thereto. For example, as a consequence of the capability negotiation in respect of a newly discovered EMR data source, the application may identify that it is compatible with the FHIR and HL7 standards, and maintains records in respect of medical records in respect of patients admitted to the emergency unit in accordance with a schema specified in the FHIR standard.

In the example set forth in Figure 4, the system 103 receives a request for healthcare data from the user device 101 or a decision support system 401. The system 103 may determine whether the requested healthcare data can be acquired on the basis of the known associations (i.e. the first plurality of associations and the second association). If the requested healthcare data can be provided on the basis of the known associations, then the system 103 retrieves the requested healthcare data on the basis of the known associations. If the requested healthcare data cannot be provided on the basis of the known associations, then the system 103 causes the application 303 to detect an association between one or more of the data sources 107 comprising the requested healthcare data and store it in the database 105, thereby causing the detected association to form part of the known associations.

In at least some examples, the system 103 may receive a further request for healthcare data from the same or different user device 101 or the support system 401 (as appropriate), wherein the requested healthcare data is stored in the second data source. In this case, the system 103 may retrieve the requested healthcare data on the basis of the known associations, which include the second association, and transmit it for receipt by the user device 101 or the decision support system 401 (as appropriate). Therefore, the system 103 adds the newly detected associations to known associations and use them for provisioning healthcare data.

In at least some examples, the system 103 may remove an association from the known associations, thereby ceasing access to all or part of healthcare data maintained in an associated data source via the healthcare network 100. The system may, for example, remove an association in response to a data source being replaced or being temporarily or permanently being taken out of service. Information regarding events that may cause an association to be permanently or temporarily removed may be acquired from the aforementioned message bus. The system 103 may, additionally or alternatively, in response to receiving data indicative of a change between the second data source and healthcare data stored therein, update the second association accordingly. Thus, the system 103 continues to facilitate access to the healthcare data stored in the second data source via the healthcare network 100.

In at least some examples, the system 103 creates one or more virtual maps based on the known associations indicative of links to the known data sources (i.e. the first plurality of data sources and the second data source) and healthcare data contained therein, thereby creating one or more patient models. As described above, a patient model may be an individual patient model, a collective patient model, a population patient model, a statistical patient model and/or a clinical patient model. In examples, the or each patient model may cause requests for healthcare data to be processed quicker because patient models map relevant healthcare data stored in disparate data sources.

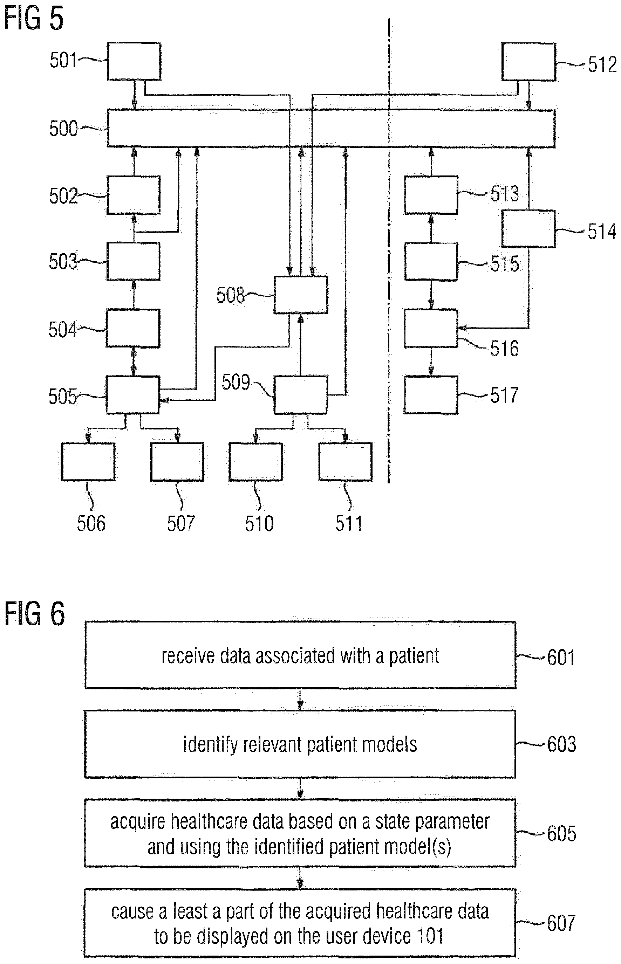

In examples, one or more of the patient models may be a schema with relevant events, use cases and/or clinical processes represented as a dependency tree. In the example set forth in Figure 5, an individual patient model 500 is represented as a dependency tree. In this example, a patient visits a healthcare professional with one or more symptoms (block 501). The healthcare professional inputs relevant notes via, for example, a user interface provided by an application on the user device 101 to be stored in one or more of the data sources 107 (block 502).

The healthcare professional may examine the patient (block 503) and may set up a diagnostic procedure involving, for example, imaging scans (such as a CT scan) and/or clinical tests (such as a blood test) (block 504). The diagnostic procedures may then be carried (blocks 506 and 507), and results may be made available to the same or another healthcare professional (block 505). Depending on the results and various assessments by healthcare professional(s), a medical condition may be identified (block 508) and a treatment plan may be created (block 509). The treatment plan in case of cancer may, for example, include surgery, chemotherapy and medication (blocks 510 and 511). The treatment plan may be revised based on how the patient is responding thereto. All the relevant information and dependencies are updated so that the patient model 500 is up-to-date.

Subsequent to conclusion of treatment, the patient may visit the same or another healthcare professional with the same or different set of symptoms (block 512) and notes regarding the visit may be entered for storage on the same or different of the data sources 107 (block 513). The healthcare professional may consult notes regarding the patient's history using the patient model 500, and may use that in addition to current symptoms for preparing a new diagnostic plan (block 515), which may then be executed at block 516 and relevant results updated at block 517. Based on the results of the diagnostic tests, an unrelated, a related or a comorbidity medical condition may be identified at block 514.

In at least some examples, the system 103 uses one or more of the patient models to identify relevant healthcare data at every stage of analysis for provisioning to a healthcare professional. This being the case, in the example set forth with reference to Figure 5, the system 103 may control the display of the user device 101 to present different healthcare data to the healthcare professional in respect of at least some of the blocks 501 to 517. For example, a healthcare professional at block 512 may be presented with medical history concerning the patient for use in identifying any comorbidities, but a healthcare professional carrying out a diagnostic procedure at block 516 may not be presented with that information as it may not be relevant for that stage. Therefore, examples retrieve relevant healthcare data for a current stage of analysis from disparate data sources and present it, and thereby reduce the cognitive burden.

In the example set forth in Figure 6, the system 103 may receive data, for example, associated with a patient and/or, a group of patients from the user device 101 or the support system 401 (block 601). The data may, for example, comprise data identifying the patient(s), symptoms, medical conditions and/or medical history. The system 103 may identify one or more of the relevant patient models (block 603), and cause the user device 101 to acquire relevant healthcare data based on a state parameter and using the identified patient model(s) (block 605). The state parameter may be indicative of one of different stages associated with analysis of the received healthcare data. For example, in the example set forth in Figure 5 the state parameter may be indicative of one of the blocks 501-517. The system 103 may cause at least a part of the acquired healthcare data to be displayed on the user device 101 (block 607). The healthcare data may, for example, be selectively displayed based on an identity of a healthcare professional, and may be anonymised prior to presentation for confidentiality.

For example, a patient may identify an unusual lump in her breast and may visit a healthcare professional, such as a general practitioner, to get it checked. The system 103 may identify an individual patient model for the patient for use in retrieving relevant healthcare data to the healthcare professional. The healthcare professional may prepare a diagnostic plan for the patient, which may include mammogram, ultrasound, biopsy, scans and/or x-rays, to be carried out by the same or different healthcare professional(s). In addition, various medical records concerning the patient may be updated to identify potential ailments, such as breast cancer. Responsive to the updates to the various medical records, one or more related patient models may be updated. For example, data indicative of the patient or their medical records may be included in a patient model for patients suspected to have breast cancer.

The system 103 may, based on the diagnostic plan, cause follow-up appointments for the patient to be scheduled. The patient may visit healthcare professional(s) for diagnostic tests, and each of the healthcare professionals may presented with different relevant healthcare data. For example, the healthcare professional conducting mammogram may be presented with information such as whether the patient has had breast implants, but this information may not be presented to a healthcare profession carrying out a blood test. Therefore, relevant healthcare data concerning the patient is presented at every stage of assessment.

Following conclusion of the diagnostic plan, it may be confirmed that the patient indeed has breast cancer. The system 103 may update the relevant patient models following conclusion of the diagnostic test. The patient may be put on a therapy plan following conclusion of the diagnostic plan, which may, for example, involve radio, chemo, hormone and/or biological therapies. The healthcare professional(s) preparing the therapy plan may be presented with relevant healthcare data. The relevant data may include available clinical resources, guidelines and regulations retrieved using, for example, a population patient model. The relevant data may, additionally or alternatively, include data regarding success rates in various similar cases retrieved using, for example, a statistical and/or a clinical patient model. The relevant data may, additionally or alternatively, include results from various diagnostic tests retrieved using, for example, an individual patient model. The system 103 may cause the relevant patient models to be updated to include data indicative of the therapy plan and/or the patient's response thereto. Following the therapy, a care plan, including counselling and/or psychological care, may be devised for the patient.

In another example, a patient with persistent cough and/or breathlessness may be prescribed a diagnostic plan for suspected lung cancer patients. In this case, the system 103 may cause relevant healthcare data, such as risk factors, including history of smoking, may be presented to the healthcare professional. The diagnostic plan may be prepared to confirm existence of lung cancer and/or its stage, and may, for example, include x-ray, scans, bronchoscopy and/or biopsy. As described above, different healthcare data may be presented to healthcare professionals at different stages. Based on the results, a therapy plan may be devised for the patient, which may, for example, include surgery, radiotherapy and/or chemotherapy.

In another example, a patient with problems relating to urinating may be prescribed a diagnostic plan for suspected prostate cancer patients. In this case, the system 103 may cause relevant healthcare data, such as risk factors, including age, ethnicity, family history, weight and diet, may be presented to the healthcare professional. The diagnostic plan may be prepared to confirm existence of prostate cancer and/or its stage, and may, for example, include PSA testing, digital rectal examination and/or biopsy. As described above, different healthcare data may be presented to healthcare professionals at different stages. Based on the results, a therapy plan may be devised for the patient, which may, for example, include surgery, radiotherapy and/or chemotherapy.

In some arrangements, the system 103 can monitor prior actions of one or more healthcare professionals when analysing healthcare data associated with at least some of the different stages. The prior actions may be monitored when, for example, the healthcare professional is analysing healthcare data in respect of the or another patient. The system 103, based on the monitoring of the prior actions and the state parameter, causes at least a part of the healthcare data acquired at block 605 to be displayed on the user device 101 in a tailored manner. Therefore, examples customise the information presented in accordance with healthcare professionals' style of working, and as a consequence are capable of personalising display of healthcare data.

The system 103 may, additionally or alternatively process the healthcare data acquired at block 605 based on the monitoring of the prior actions. For example, if the system 103 determines that a healthcare professional changes the scanned images to a certain resolution, then the system 103 may process subsequently received scanned images to that same resolution when presenting them to the healthcare professional. The system 103 may then cause at least part of processed healthcare data to be displayed on the user device 101.

In at least some examples, the system 103, in response to receiving a user input, for example, from a healthcare professional via the user device 101 or the support system 401, may cause the user device 101 or the support system 401 to acquire further healthcare data based on a combination of the received user input and the state parameter, and using the patient model(s) identified at block 603. The system 103 may cause the user device 101 to present at least a part of the further healthcare data. Therefore, examples update relevant healthcare data based on the healthcare professional's interaction in connection with the healthcare data that is currently presented.

In at least some examples, the system 103 may cause the user device 101 to process at least a part of the healthcare data acquired at block 605 based on a predetermined parameter, and thereafter causes at least a part of the processed healthcare data to be displayed on the user device 101. The predetermined parameter may, for example, comprise or be based on: data associated with the patient usable for processing, for example, allergies; data associated with another patient usable for processing, for example, similarities and/or comorbidities; data indicative of a healthcare user or professional usable for processing healthcare data in accordance with, for example, personal preferences; data indicative of a medical condition usable for processing healthcare data related to, for example, statistical or clinical decision support; data indicative of one or more guidelines usable for processing healthcare data to comply with, for example, relevant guidelines; data indicative of one or more legal regulations usable for processing healthcare data in accordance with, for example, relevant legislations; data indicative of a customisation parameter usable for processing healthcare data in accordance with, for example, local customisations; and/or data indicative of a user setting usable for processing healthcare data in accordance with, for example, user settings. Therefore, examples pre-process acquired healthcare data and thereby reduce the burden.

In at least some examples, the system 103 may maintain a context parameter indicative of a clinical step associated with the healthcare data received at block 601. For example, the context parameter in case of a patient undergoing bronchoscopy may be indicative of a current clinical step in the procedure: premedication, sedation, administration of oxygen, brushing, biopsy, etc. The system 103 may filter the healthcare data acquired at block 605 based on the context parameter, and cause at least a part of the filtered healthcare data to be displayed on the user device 101 or the support system 401. In examples, the healthcare data may, additionally or alternatively, be filtered based on: a medical condition or disease; the patient being examined; a healthcare profession assessing the information presented; a current stage of assessment; and/or a clinical path. Filtering may be performed in stages, such as filtering based on medical condition followed by data specific to the patient being examined. Therefore, examples present relevant healthcare data for a current step in a clinical process by filtering out irrelevant information.

The system 103 may control display of at least part of the filtered healthcare data to be displayed on the user device 101 or the support system 401 based on a combination of the context parameter and the state parameter. For example, the state parameter may be indicative of a clinical process, such as treatment stage bronchoscopy, and the context parameter may be indicative of the current step in the procedure, such as premedication. The system 103 may, additionally or alternatively, customise the information presented in accordance with an associated frame of reference, such as a healthcare professional associated with a current stage. For example, a comorbidity for a lung cancer patient can be diabetes, so it is important that this information is presented to a healthcare profession generating a treatment or diagnosis plan but not as important for a healthcare professional carrying out a diagnostic test in accordance with the diagnostic plan.

In examples, the system 103 may link the healthcare data acquired at block 605, and present a pictorial or graphical representation thereof, thereby reducing the burden. The representation may, for example, be in the form of a timeline, a clinical path, a decision tree and/or a dependency tree.

In some arrangements, the healthcare data is acquired at block 605 based on: data indicative of a patient; data indicative of a healthcare user or professional; data indicative of a medical condition; data indicative of one or more guidelines; data indicative of one or more legal regulations; data indicative of a customisation parameter; and/or data indicative of a user setting. Therefore, examples acquire relevant healthcare data based on associated frame of reference and/or user preference.

In examples, the healthcare data acquired at block 605 comprises data indicative of: healthcare data associated with a patient being examined; healthcare data associated with one or more further patients; and/or expert medical knowledge. The system 103 may, for example, cause further relevant healthcare data related to the patient to be acquired to aid analysis and/or for analysis in relation to a comorbidity. The system 103 may, additionally or alternatively, retrieve healthcare data corresponding to other patients with similar symptoms and/or conditions. The system 103 may, additionally or alternatively, retrieve expert healthcare data from, for example, scientific journals to aid a healthcare professional. Therefore, examples provide comprehensive healthcare data at every stage.

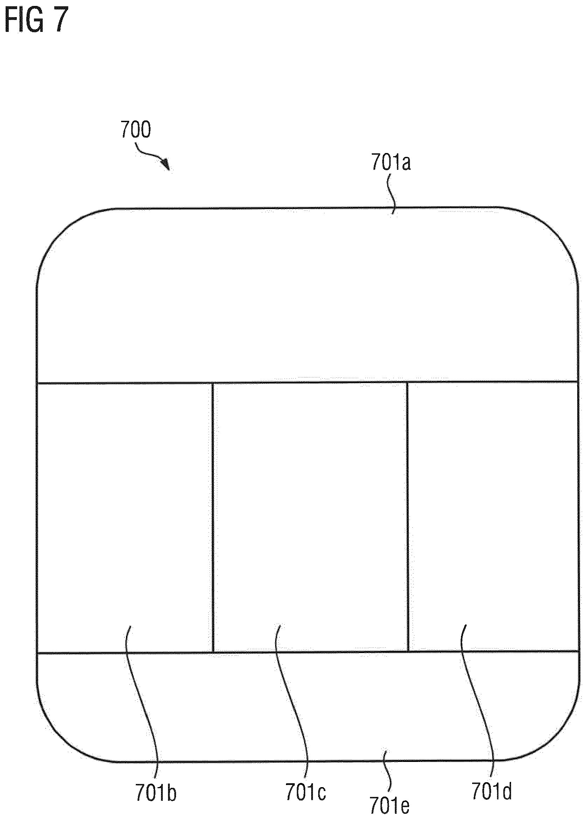

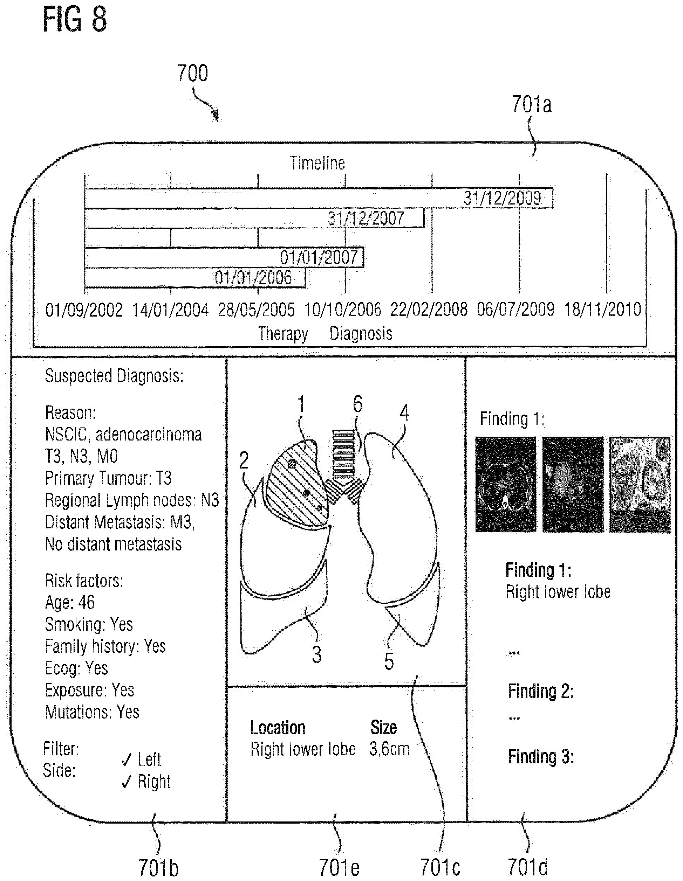

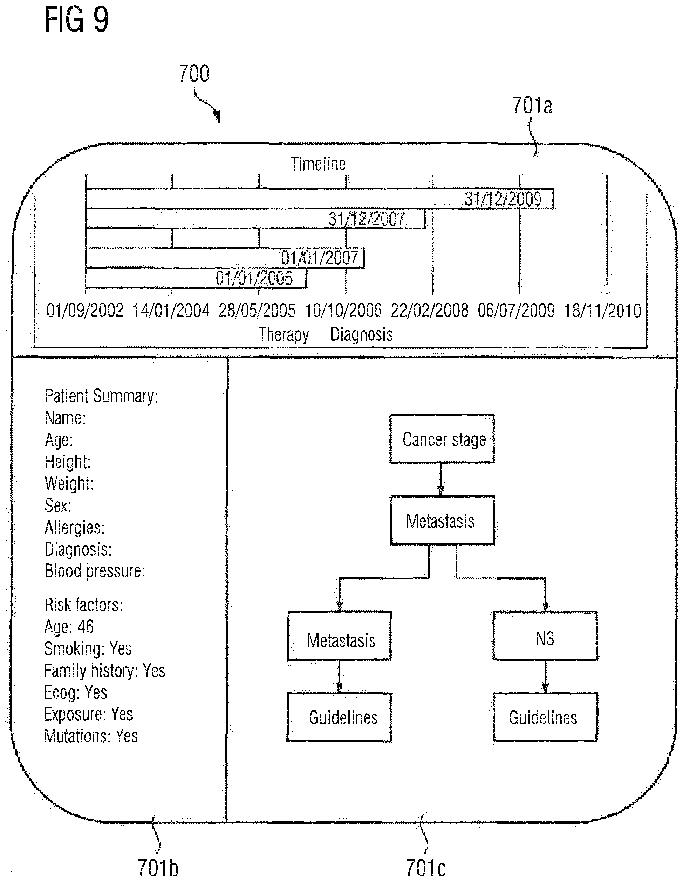

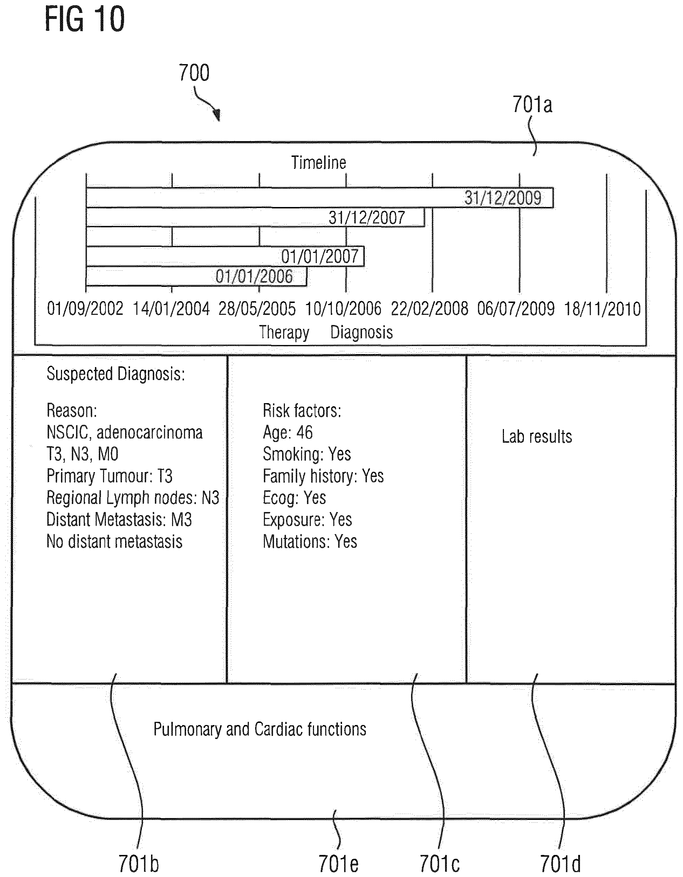

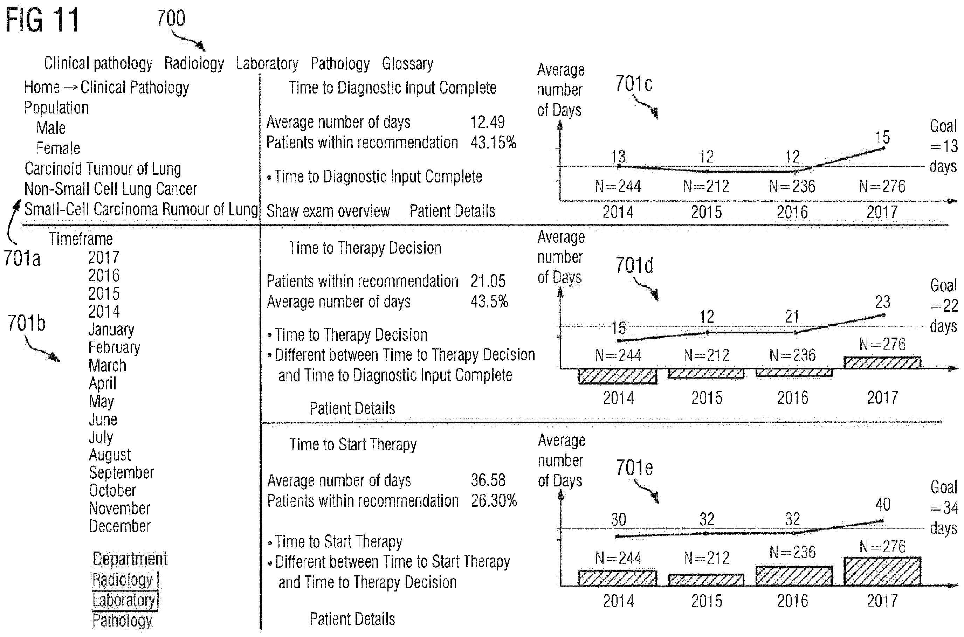

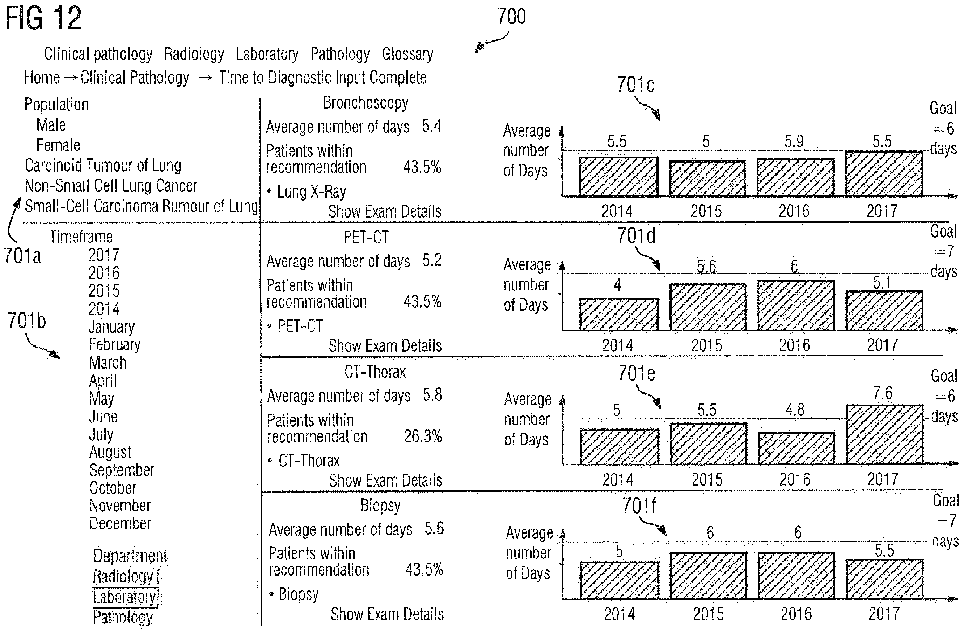

In the example set forth in Figure 7, an interface 700 provided by an application on the user device 101 for presentation of healthcare data acquired at block 605 or otherwise acquired. The healthcare data may correspond to one or more patients, and may be presented for use in making clinical, administrative and/or management decisions.

In this example, the interface 700 is split into zones 701a-e for presenting different types of healthcare data. Healthcare data may be organised in the zones based on user customisations and/or system settings. For example, the system 103 may cause a timeline indicative of a patient's medical history or a clinical path to be generated based on the healthcare data acquired at block 605 and presented in the zone 701a to present an overview their medical history to a healthcare professional.

In the zone 701b the system 103 may cause the interface 700 to present symptoms being experienced and other metadata, such as age, risks and allergies, related to the patient.

In the zone 701c the system 103 may cause the interface 700 to present a pictorial representation of the affected organs and regions thereof, and/or images from scans conducted during the diagnostic stage. For example, in respect of a patient with brain tumour the system 103 may cause an image of a brain identifying location of one or more tumours to be displayed in the zone 701c. The system 103 may, additionally or alternatively, cause a relevant dependency or decision tree to be displayed in the zone 701c to aid a healthcare professional with his assessment

In the zone 701d the system 103 may cause the interface 700 to present relevant information from a statistical and/or a clinical decision support system.

In the zone 701e the system 103 may cause the interface 700 to present relevant healthcare data based on the aforementioned context parameter, such as data identifying a healthcare professional and/or a current stage of analysis.

In the example set forth in Figure 7a, the user interface 700 is customised for a healthcare professional devising a therapy plan for a patient with lung cancer in the right lower lobe. In this example, the healthcare professional is presented with a timeline in the zone 701a setting out time periods associated with the condition and the treatment process. In examples, the zone 701a may, additionally or alternatively, include links to other parts of the interface 700 configured to present related healthcare information, such as status of the treatment plan, diagnostic procedures and their results, and details regarding the therapy plan. In examples, the zone 701a may, additionally or alternatively, include a link to the healthcare professional's other patients, appointments diary and/or relevant guidelines and regulations.

In the zone 701b, relevant healthcare data, such as details of the diagnosis and risk factors may be presented. In addition, may include means for filtering the presented information, such as check boxes that enable the healthcare professional to limit the information displayed, such as limiting healthcare data to right and/or left side of the lung.

In the zone 701c, a pictorial representation of a lung identifying location of the cancer may be displayed. In the zone 701d, data indicative of the various diagnostic tests and/or their findings may be displayed. In the zone 701e relevant metadata regarding the cancer, such as type, size and location may be displayed.

In the example set forth in Figure 7b, the user interface 700 is customised to display status information regarding a case involving a patient with lung cancer. In this example, the timeline indicative of the clinical process is displayed in the zone 701a. In the zone 701b, relevant metadata related to the patient is displayed along with the risk factors. In the zone 701c a workflow representing various clinical stages is displayed along with links to guidelines relevant for a current stage of examination.

In the example set forth in Figure 7c, the user interface 700 is customised to display status information regarding a case involving a patient with lung cancer. In this example, the timeline indicative of the clinical process is displayed in the zone 701a. In the zone 701b, relevant metadata related to the patient is displayed. In the zone 701c risk factors associated with the patient are displayed. In the zone 701d, data indicative of results from the various lab and diagnostic tests are displayed. In the zone 701e data indicative of the status of the therapies, such as concomitant cardiac and pulmonary operations, are displayed.

In the example set forth in Figure 7d, the user interface 700 is customised to display data indicative of population health management for use in making administrative and/or managerial decisions, and/or monitoring performance. In this example, various metrics relating to lung cancer patients are displayed. The user interface 700 includes means for controlling information displayed in the various zones 701, such as links, at the top, and these include limiting information relevant to "Clinical Pathology", "Radiology", "Laboratory", and "Pathology". The links may additionally include pointers to reference information, such as a link to "Glossary".

In this example, information relevant to "Clinical Pathology" is displayed in the various zones 701. In the zone 701a metadata relating to a proportion of patient population selected for use in preparing the various displayed metrics. The zone 701b includes means for restricting the proportion of patient population selected for use in preparing the various displayed metrics, for example, by restricting the timeframe for assessment. For example, the healthcare professional may restrict the displayed metrics patients being treated in the year 2017.

The user interface 700 may present metadata and a graph relating to each of the metrics selected for display in the zones 701c-e. In this example, the zone 701c displays metadata and a graph relating to average time taken for the clinical stage "TIME TO DIAGNOSTIC INPUT COMPLETE", the zone 701d displays metadata and a graph relating to average time taken for the clinical stage "TIME TO THERAPY DECISION", and the zone 701e displays metadata and a graph relating to average time taken for the clinical stage "TIME TO START OF THERAPY". Management of the healthcare network 100 may, for example, use this information for assessing whether key targets have been met. Therefore, the user interface 700 is customised based on healthcare professionals' role and task.

In the example set forth in Figure 7e, the user interface 700 is customised to display data indicative of population health management for use in making administrative and/or managerial decisions, and/or monitoring performance. In this example, various metrics relating to lung cancer patients are displayed. Similar to the example set forth in Figure 7e, the user interface 700 includes means for controlling information displayed in the various zones 701, such as links, at the top, and these include limiting information relevant to "Clinical Pathology", "Radiology", "Laboratory", and "Pathology". The links may additionally include pointers to reference information, such as a link to "Glossary".

In this example, information relevant to "Clinical Pathology" is displayed in the various zones 701, specifically information relating to the various diagnostic procedures in respect of lung cancer. In examples, the healthcare professional may navigate to the display in accordance with Figure 7e from one or more links presented in the example set forth in Figure 7d, such as the link "Laboratory".

In this example, in the zone 701a metadata relating to a proportion of patient population selected for use in preparing the various displayed metrics. The zone 701b includes means for restricting the proportion of patient population selected for use in preparing the various displayed metrics, for example, by restricting the timeframe for assessment. For example, the healthcare professional may restrict the displayed metrics patients being treated in the year 2017.

The user interface 700 may present metadata and a graph relating to each of the metrics selected for display in the zones 701c-f. In this example, the zone 701c displays metadata and a graph relating to average time taken for the diagnostic procedure "Bronchoscopy", the zone 701d displays metadata and a graph relating to average time taken for the diagnostic procedure "PET-CT", the zone 701e displays metadata and a graph relating to average time taken for the diagnostic procedure "CT-Thorax", and the zone 701f displays metadata and a graph relating to average time taken for the diagnostic procedure "Biopsy". Management of the healthcare network 100 may, for example, use this information for resource management.

Therefore, the framework enables a homogeneous environment in which links to disparate data sources are maintained and updated based on information required, thereby integrating disparate data sources. The retrieved information is based on relevant context, and is automatically updated as the analysis advances. In addition, presented information is customised in accordance with preferences of healthcare professionals', thereby improving user experience.

The above are to be understood as illustrative examples. Further examples are envisaged.

In examples described above, the healthcare network 100 comprises one or more user devices 101, a system 103, a database 105 and one or more data sources 107. In other examples, the healthcare network comprises additional systems.

## Claims

1. A system (103) operable to provide healthcare data to a user device (101) in a healthcare network (100), the healthcare network (100) comprising a plurality of data sources (107) comprising healthcare data, the system comprising at least one processor and at least one memory including computer program code, the at least one memory and the computer program code configured to, with the at least one processor, cause the system (103) at least to perform the steps of:
maintaining, in a database (105), a first plurality of associations between a first plurality of the data sources and healthcare data stored therein;
receiving, from the user device (101), a request for first healthcare data, wherein the requested first healthcare data is stored in a second of the data sources (107), the second data source being different to the first plurality of data sources;
determining whether the requested first healthcare data can be retrieved based on the first plurality of associations;
selectively, based on an outcome of the determining, causing a second association between the second data source and the requested first healthcare data to be detected and stored in the database (105);
retrieving the requested first healthcare data from the second data source; and
transmitting the retrieved first healthcare data for receipt by the user device (101).

2. A system (103) according to claim 1, wherein the at least one memory and the computer program code are configured to, with the at least one processor, cause the system (103) to perform the step of:
causing the second association to be detected if an association corresponding to the requested first healthcare data is not determined on the basis of the first plurality of associations.

3. A system (103) according to claims 1 or 2, wherein the at least one memory and the computer program code are configured to, with the at least one processor, cause the system (103) to perform the steps of:
receiving, from the user device (101), a request for second healthcare data, wherein the second healthcare data is stored in a data source of the first plurality of data sources;
determining that the second healthcare data can be retrieved based on an association of the first plurality of associations;
retrieving the requested second healthcare data on the basis of the determined association; and
transmitting the retrieved second healthcare data for receipt by the user device (101).

4. A system (103) according to any of claims 1 to 3, wherein the at least one memory and the computer program code are configured to, with the at least one processor, cause the system (103) to perform the step of:
using an application (303) to detect which of the data sources (107) comprises the requested healthcare data, thereby detecting the second association.

5. A system according to claim 4, wherein the application (303) comprises crawling functionality.

6. A system (103) according to any of claims 1 to 5, wherein the at least one memory and the computer program code are configured to, with the at least one processor, cause the system (103) to perform the steps of:
receiving, from the user device (101), a further request for healthcare data, wherein the further requested healthcare data is stored in the second data source;
retrieving the further requested healthcare data on the basis of the second association; and
transmitting the retrieved further requested healthcare data for receipt by the user device (101).

7. A system (103) according to any of claims 1 to 6, wherein the at least one memory and the computer program code are configured to, with the at least one processor, cause the system (103) to perform the step of:
removing an association from the first plurality of associations, thereby ceasing access to the corresponding healthcare data via the healthcare network (100).

8. A system (103) according to any of claims 1 to 7, wherein the at least one memory and the computer program code are configured to, with the at least one processor, cause the system (103) to perform the step of:
in response to receiving data indicative of a change between the second data source and healthcare data stored therein, updating the second association, thereby continuing to facilitate access to the healthcare data stored in the second data source via the healthcare network (100).

9. A system (103) according to any of claims 1 to 8, wherein the at least one memory and the computer program code are configured to, with the at least one processor, cause the system (103) to perform the step of:
creating a virtual map of the first plurality of data sources and the second data source based on the first plurality of associations and the second association, thereby creating a patient model (500).

10. A system (103) according to claim 9, wherein the at least one memory and the computer program code are configured to, with the at least one processor, cause the system (103) to perform the steps of:
in response to receiving data associated with a patient, causing the user device (101) to acquire healthcare data based on a state parameter and using the patient model (500), the state parameter being indicative of one of different stages (501-517) associated with analysis of the received data; and
causing at least a part of the acquired healthcare data to be displayed on the user device (101).

11. A system (103) according to claim 10, wherein the at least one memory and the computer program code are configured to, with the at least one processor, cause the system (103) to perform the steps of:
monitoring prior actions of a user when analysing healthcare data associated with at least some of the different stages (501-517); and
controlling display of at least a part of the acquired healthcare data on the user device (101) based on a combination of the state parameter and the monitoring.

12. A system (103) according to claims 10 or 11, wherein the at least one memory and the computer program code are configured to, with the at least one processor, cause the system (103) to perform the steps of:
in response to a user input, causing the user device (101) to acquire further healthcare data based on a combination of the input and the state parameter, and using the patient model (500); and
causing the user device (101) to present at least a part of the acquired further healthcare data.

13. A system (103) according to any of claims 10 to 12, wherein the at least one memory and the computer program code are configured to, with the at least one processor, cause the system (103) to perform the steps of:
cause the user device (101) to process the acquired healthcare data based on a predetermined parameter; and
causing at least a part of the processed healthcare data to be displayed on the user device (101).

14. A system (103) according to claim 13, wherein the predetermined parameter comprises:
data associated with the patient;
data associated with another patient;
data indicative of a healthcare user;
data indicative of a medical condition;
data indicative of one or more guidelines;
data indicative of one or more legal regulations;
data indicative of a customisation parameter; and/or
data indicative of a user setting.

15. A system (103) according to any of claims 10 to 14, wherein the at least one memory and the computer program code are configured to, with the at least one processor, cause the system to perform the steps of:
maintaining a context parameter indicative of a clinical step associated with analysis of the received data;
filtering the acquired healthcare data based on the context parameter; and
causing at least a part of the filtered healthcare data to be displayed on the user device (101).

16. A system (103) according to claim 15, wherein the at least one memory and the computer program code are configured to, with the at least one processor, cause the system (103) to perform the step of:
controlling display of at least a part of the filtered healthcare data on the user device (101) based on a combination of the state parameter and the context parameter.

17. A system (103) according to any of claims 10 to 16, wherein the healthcare data is acquired based on:
data indicative of the patient;
data indicative of a healthcare user;
data indicative of a medical condition;
data indicative of one or more guidelines;
data indicative of one or more legal regulations;
data indicative of a customisation parameter; and/or
data indicative of a user setting.

18. A system (103) according to any of claims 10 to 17, wherein the acquired healthcare data comprises data indicative of:
healthcare data associated with the patient;
healthcare data associated with another patient; and/or
expert medical knowledge.

19. A system (103) according to any of claims 1 to 18, wherein the data sources (107) comprise:
an Electronic Medical Records (EMR) data source;
a Picture Archiving Communication Systems (PACS) data source;
an Emergency Care Summary (ECS) data source;
a Key Information Summary (KIS) data source;
an Electronic Healthcare Record (EHR) data source;
an Electronic Patient Record (EPR) data source; and/or
a Personal Health Record (PHR) data source.

20. A system (103) according to any of claims 1 to 19, wherein the system (103) is configured to communicate with a first of the data sources (107) using an application programming interface (305) in accordance with a Fast Healthcare Interoperability Resources (FHIR) standard.

21. A method of providing healthcare data to a user device (101) in a healthcare network (100), the healthcare network (100) comprising a plurality of data sources (107) comprising healthcare data, the method comprising:
maintaining, in a database (105), a first plurality of associations between a first plurality of the data sources and healthcare data stored therein;
receiving, from the user device (101), a request for first healthcare data, wherein the requested first healthcare data is stored in a second of the data sources (107), the second data source being different to the first plurality of data sources;
determining whether the requested first healthcare data can be retrieved based on the first plurality of associations;
selectively, based on an outcome of the determining, causing a second association between the second data source and the requested first healthcare data to be detected and stored in the database (105);
retrieving the requested first healthcare data from the second data source; and
transmitting the retrieved first healthcare data for receipt by the user device (101).

22. A computer program comprising a set of instructions, which, when executed by a computerised device, cause the computerised device to perform a method of providing healthcare data to a user device (101) in a healthcare network (100), the healthcare network comprising a plurality of data sources comprising healthcare data, the method comprising, at the computerised device:
maintaining, in a database (105), a first plurality of associations between a first plurality of the data sources and healthcare data stored therein;
receiving, from the user device (101), a request for first healthcare data, wherein the requested first healthcare data is stored in a second of the data sources (107), the second data source being different to the first plurality of data sources;
determining whether the requested first healthcare data can be retrieved based on the first plurality of associations;
selectively, based on an outcome of the determining, causing a second association between the second data source and the requested first healthcare data to be detected and stored in the database;
retrieving the requested first healthcare data from the second data source; and
transmitting the retrieved first healthcare data for receipt by the user device (101).

## Patentansprüche

1. System (103), das betreibbar ist, um Gesundheitsversorgungsdaten für eine Benutzervorrichtung (101) in einem Gesundheitsversorgungsnetz (100) bereitzustellen, wobei das Gesundheitsversorgungsnetz (100) eine Vielzahl von Datenquellen (107) umfasst, die Gesundheitsversorgungsdaten umfassen, wobei das System wenigstens einen Prozessor und wenigstens einen Speicher, der Computerprogrammcode enthält, umfasst, wobei der wenigstens eine Speicher und der Computerprogrammcode dafür ausgelegt sind, zusammen mit dem wenigstens einen Prozessor das System (103) zu veranlassen, wenigstens die folgenden Schritte durchzuführen:
Pflegen, in einer Datenbank (105), einer ersten Vielzahl von Assoziationen zwischen einer ersten Vielzahl von Datenquellen und darin gespeicherten Gesundheitsversorgungsdaten;
Empfangen, von der Benutzervorrichtung (101), einer Anforderung von ersten Gesundheitsversorgungsdaten, wobei die angeforderten ersten Gesundheitsversorgungsdaten in einer zweiten der Datenquellen (107) gespeichert sind, wobei die zweite Datenquelle von der ersten Vielzahl von Datenquellen verschieden ist;
Bestimmen, ob die angeforderten ersten Gesundheitsversorgungsdaten abgerufen werden können, basierend auf der ersten Vielzahl von Assoziationen;
selektiv, basierend auf einem Ergebnis des Bestimmens, bewirken, dass eine zweite Assoziation zwischen der zweiten Datenquelle und den angeforderten ersten Gesundheitsversorgungsdaten erkannt und in der Datenbank (105) gespeichert wird;
Abrufen der angeforderten ersten Gesundheitsversorgungsdaten von der zweiten Datenquelle; und
Übertragen der abgerufenen ersten Gesundheitsversorgungsdaten zum Empfang durch die Benutzervorrichtung (101).

2. Verfahren (103) gemäß Anspruch 1, wobei der wenigstens eine Speicher und der Computerprogrammcode dafür ausgelegt sind, zusammen mit dem wenigstens einen Prozessor das System (103) zu veranlassen, den folgenden Schritt durchzuführen:
Bewirken, dass die zweite Assoziation erkannt wird, wenn eine Assoziation, die den angeforderten ersten Gesundheitsversorgungsdaten entspricht, nicht bestimmt wird, basierend auf der ersten Vielzahl von Assoziationen.

3. System (103) gemäß Anspruch 1 oder 2, wobei der wenigstens eine Speicher und der Computerprogrammcode dafür ausgelegt sind, zusammen mit dem wenigstens einen Prozessor das System (103) zu veranlassen, die folgenden Schritte durchzuführen:
Empfangen, von der Benutzervorrichtung (101), einer Anforderung von zweiten Gesundheitsversorgungsdaten, wobei die zweiten Gesundheitsdaten in einer Datenquelle der ersten Vielzahl von Datenquellen gespeichert sind;
Bestimmen, dass die zweiten Gesundheitsversorgungsdaten abgerufen werden können, basierend auf einer Assoziation der ersten Vielzahl von Assoziationen;
Abrufen der angeforderten zweiten Gesundheitsversorgungsdaten basierend auf der bestimmten Assoziation; und
Übertragen der abgerufenen zweiten Gesundheitsversorgungsdaten zum Empfang durch die Benutzervorrichtung (101).

4. System (103) gemäß einem der Ansprüche 1 bis 3, wobei der wenigstens eine Speicher und der Computerprogrammcode dafür ausgelegt sind, zusammen mit dem wenigstens einen Prozessor das System (103) zu veranlassen, den folgenden Schritt durchzuführen:
Verwenden einer Anwendung (303), um zu erkennen, welche der Datenquellen (107) die angeforderten Gesundheitsversorgungsdaten umfasst, wodurch die zweite Assoziation erkannt wird.

5. System gemäß Anspruch 4, wobei die Anwendung (303) eine Crawling-Funktionalität umfasst.

6. System (103) gemäß einem der Ansprüche 1 bis 5, wobei der wenigstens eine Speicher und der Computerprogrammcode dafür ausgelegt sind, zusammen mit dem wenigstens einen Prozessor das System (103) zu veranlassen, die folgenden Schritte durchzuführen:
Empfangen, von der Benutzervorrichtung (101), einer weiteren Anforderung von Gesundheitsversorgungsdaten, wobei die weiteren angeforderten Gesundheitsversorgungsdaten in der zweiten Datenquelle gespeichert sind;
Abrufen der weiteren angeforderten Gesundheitsversorgungsdaten basierend auf der zweiten Assoziation; und
Übertragen der abgerufenen weiteren angeforderten Gesundheitsversorgungsdaten zum Empfang durch die Benutzervorrichtung (101).

7. System (103) gemäß einem der Ansprüche 1 bis 6, wobei der wenigstens eine Speicher und der Computerprogrammcode dafür ausgelegt sind, zusammen mit dem wenigstens einen Prozessor das System (103) zu veranlassen, den folgenden Schritt durchzuführen:
Entfernen einer Assoziation aus der ersten Vielzahl von Assoziationen, wodurch der Zugriff auf die entsprechenden Gesundheitsversorgungsdaten über das Gesundheitsversorgungsnetz (100) eingestellt wird.

8. System (103) gemäß einem der Ansprüche 1 bis 7, wobei der wenigstens eine Speicher und der Computerprogrammcode dafür ausgelegt sind, zusammen mit dem wenigstens einen Prozessor das System (103) zu veranlassen, den folgenden Schritt durchzuführen:
als Reaktion auf das Empfangen von Daten, die auf eine Änderung zwischen der zweiten Datenquelle und den darin gespeicherten Gesundheitsversorgungsdaten hinweisen,
Aktualisieren der zweiten Assoziation und dadurch Ermöglichen des fortgesetzten Zugriffs auf die in der zweiten Datenquelle gespeicherten Gesundheitsversorgungsdaten über das Gesundheitsversorgungsnetz (100).

9. System (103) gemäß einem der Ansprüche 1 bis 8, wobei der wenigstens eine Speicher und der Computerprogrammcode dafür ausgelegt sind, zusammen mit dem wenigstens einen Prozessor das System (103) zu veranlassen, den folgenden Schritt durchzuführen:
Erstellen einer virtuellen Karte der ersten Vielzahl von Datenquellen und der zweiten Datenquelle basierend auf der ersten Vielzahl von Assoziationen und der zweiten Assoziation, wodurch ein Patientenmodell (500) erstellt wird.

10. System (103) gemäß Anspruch 9, wobei der wenigstens eine Speicher und der Computerprogrammcode dafür ausgelegt sind, zusammen mit dem wenigstens einen Prozessor das System (103) zu veranlassen, die folgenden Schritte durchzuführen:
als Reaktion auf das Empfangen von Daten, die mit einem Patienten verknüpft sind, Veranlassen der Benutzervorrichtung (101), Gesundheitsversorgungsdaten basierend auf einem Zustandsparameter und unter Verwendung des Patientenmodells (500) zu erfassen, wobei der Zustandsparameter eine von verschiedenen Stufen (501-517) anzeigt, die mit der Analyse der empfangenen Daten verknüpft sind; und
Bewirken, dass wenigstens ein Teil der erfassten Gesundheitsversorgungsdaten auf der Benutzervorrichtung (101) angezeigt wird.

11. System (103) gemäß Anspruch 10, wobei der wenigstens eine Speicher und der Computerprogrammcode dafür ausgelegt sind, zusammen mit dem wenigstens einen Prozessor das System (103) zu veranlassen, die folgenden Schritte durchzuführen:
Überwachen früherer Aktionen eines Benutzers beim Analysieren von Gesundheitsversorgungsdaten, die mit wenigstens einigen der verschiedenen Stufen (501-517) verknüpft sind; und
Steuern der Anzeige wenigstens eines Teils der erfassten Gesundheitsversorgungsdaten auf der Benutzervorrichtung (101) basierend auf einer Kombination aus dem Zustandsparameter und der Überwachung.

12. System (103) gemäß Anspruch 10 oder 11, wobei der wenigstens eine Speicher und der Computerprogrammcode dafür ausgelegt sind, zusammen mit dem wenigstens einen Prozessor das System (103) zu veranlassen, die folgenden Schritte durchzuführen:
als Reaktion auf eine Benutzereingabe, Veranlassen der Benutzervorrichtung (101), weitere Gesundheitsversorgungsdaten zu erfassen, basierend auf einer Kombination aus der Eingabe und dem Zustandsparameter und unter Verwendung des Patientenmodells (500); und
Veranlassen der Benutzervorrichtung (101), wenigstens einen Teil der erfassten weiteren Gesundheitsversorgungsdaten zu präsentieren.

13. System (103) gemäß einem der Ansprüche 10 bis 12, wobei der wenigstens eine Speicher und der Computerprogrammcode dafür ausgelegt sind, zusammen mit dem wenigstens einen Prozessor das System (103) zu veranlassen, die folgenden Schritte durchzuführen:
Veranlassen der Benutzervorrichtung (101), die erfassten Gesundheitsversorgungsdaten basierend auf einem vorgegebenen Parameter zu verarbeiten; und
Bewirken, dass wenigstens ein Teil der verarbeiteten Gesundheitsversorgungsdaten auf der Benutzervorrichtung (101) angezeigt wird.

14. System (103) gemäß Anspruch 13, wobei der vorbestimmte Parameter umfasst:
mit dem Patienten verknüpfte Daten;
mit einem anderen Patienten verknüpfte Daten;
Daten, die einen Nutzer der Gesundheitsversorgung anzeigen;
Daten, die einen medizinischen Zustand anzeigen;
Daten, die eine oder mehrere Richtlinien anzeigen;
Daten, die eine oder mehrere rechtliche Vorschriften anzeigen;
Daten, die einen Parameter zur individuellen Anpassung anzeigen; und/oder
Daten, die eine Benutzereinstellung anzeigen.

15. System (103) gemäß einem der Ansprüche 10 bis 14, wobei der wenigstens eine Speicher und der Computerprogrammcode dafür ausgelegt sind, zusammen mit dem wenigstens einen Prozessor das System zu veranlassen, die folgenden Schritte durchzuführen:
Pflegen eines Kontextparameters, der einen klinischen Schritt anzeigt, der mit der Analyse der empfangenen Daten verknüpft ist;
Filtern der erfassten Gesundheitsversorgungsdaten basierend auf dem Kontextparameter; und
Bewirken, dass wenigstens ein Teil der gefilterten Gesundheitsversorgungsdaten auf der Benutzervorrichtung (101) angezeigt wird.

16. System (103) gemäß Anspruch 15, wobei der wenigstens eine Speicher und der Computerprogrammcode dafür ausgelegt sind, zusammen mit dem wenigstens einen Prozessor das System (103) zu veranlassen, den folgenden Schritt durchzuführen:
Steuern der Anzeige wenigstens eines Teils der gefilterten Gesundheitsversorgungsdaten auf der Benutzervorrichtung (101) basierend auf einer Kombination aus dem Zustandsparameter und dem Kontextparameter.

17. System (103) gemäß einem der Ansprüche 10 bis 16, wobei die Gesundheitsversorgungsdaten erfasst werden basierend auf:
Daten, die den Patienten anzeigen;
Daten, die einen Nutzer der Gesundheitsversorgung anzeigen;
Daten, die einen medizinischen Zustand anzeigen;
Daten, die eine oder mehrere Richtlinien anzeigen;
Daten, die eine oder mehrere rechtliche Vorschriften anzeigen;
Daten, die einen Parameter zur individuellen Anpassung anzeigen; und/oder
Daten, die eine Benutzereinstellung anzeigen.

18. System (103) gemäß einem der Ansprüche 10 bis 17, wobei die erfassten Gesundheitsversorgungsdaten Daten umfassen, die anzeigen:
Gesundheitsversorgungsdaten, die mit dem Patienten verknüpft sind;
Gesundheitsversorgungsdaten, die mit einem anderen Patienten verknüpft sind; und/oder
medizinisches Fachwissen.

19. System (103) gemäß einem der Ansprüche 1 bis 18, wobei die Datenquellen (107) umfassen:
eine Datenquelle für elektronische Krankenakten (Electronic Medical Records, EMR);
eine Datenquelle eines Bildarchivierungs- und Kommunikationssystems (Picture Archiving Communication System, PACS)
eine Datenquelle für eine Notfalldatenübersicht (Emergency Care Summary, ECS);
eine Datenquelle für eine Schlüsseldatenübersicht (Key Information Summary, KIS);
eine Datenquelle für eine elektronische Gesundheitsversorgungsakte (Electronic Healthcare Record, EHR) ;
eine Datenquelle für eine elektronische Patientenakte (Electronic Patient Record, EPR); und/oder
eine Datenquelle für einen persönlichen Gesundheitsdatensatz (Personal Health Record, PHR).

20. System (103) gemäß einem der Ansprüche 1 bis 19, wobei das System (103) dafür ausgelegt ist, mit einer ersten der Datenquellen (107) zu kommunizieren, unter Verwendung einer Anwendungsprogrammierschnittstelle (305) gemäß einem Standard für Ressourcen für die schnelle Zusammenarbeit in der Gesundheitsversorgung (Fast Healthcare Interoperability Resources, FHIR).

21. Verfahren zum Bereitstellen von Gesundheitsversorgungsdaten für eine Benutzervorrichtung (101) in einem Gesundheitsversorgungsnetz (100), wobei das Gesundheitsversorgungsnetz (100) eine Vielzahl von Datenquellen (107) umfasst, die Gesundheitsversorgungsdaten umfassen, wobei das Verfahren umfasst:
Pflegen, in einer Datenbank (105), einer ersten Vielzahl von Assoziationen zwischen einer ersten Vielzahl von Datenquellen und darin gespeicherten Gesundheitsversorgungsdaten;
Empfangen, von der Benutzervorrichtung (101), einer Anforderung von ersten Gesundheitsversorgungsdaten, wobei die angeforderten ersten Gesundheitsversorgungsdaten in einer zweiten der Datenquellen (107) gespeichert sind, wobei die zweite Datenquelle von der ersten Vielzahl von Datenquellen verschieden ist;
Bestimmen, ob die angeforderten ersten Gesundheitsversorgungsdaten abgerufen werden können, basierend auf der ersten Vielzahl von Assoziationen;
selektiv, basierend auf einem Ergebnis des Bestimmens, bewirken, dass eine zweite Assoziation zwischen der zweiten Datenquelle und den angeforderten ersten Gesundheitsversorgungsdaten erkannt und in der Datenbank (105) gespeichert wird;
Abrufen der angeforderten ersten Gesundheitsversorgungsdaten von der zweiten Datenquelle; und
Übertragen der abgerufenen ersten Gesundheitsversorgungsdaten zum Empfang durch die Benutzervorrichtung (101).

22. Computerprogramm, einen Satz von Anweisungen umfassend, die bei Ausführung durch eine computerisierte Vorrichtung die computerisierte Vorrichtung veranlassen, ein Verfahren zum Bereitstellen von Gesundheitsversorgungsdaten an eine Benutzervorrichtung (101) in einem Gesundheitsversorgungsnetz (100) durchzuführen, wobei das Gesundheitsversorgungsnetz eine Vielzahl von Datenquellen umfasst, die Gesundheitsversorgungsdaten umfassen, wobei das Verfahren an der computerisierten Vorrichtung umfasst:
Pflegen, in einer Datenbank (105), einer ersten Vielzahl von Assoziationen zwischen einer ersten Vielzahl von Datenquellen und darin gespeicherten Gesundheitsversorgungsdaten;
Empfangen, von der Benutzervorrichtung (101), einer Anforderung von ersten Gesundheitsversorgungsdaten, wobei die angeforderten ersten Gesundheitsversorgungsdaten in einer zweiten der Datenquellen (107) gespeichert sind, wobei die zweite Datenquelle von der ersten Vielzahl von Datenquellen verschieden ist;
Bestimmen, ob die angeforderten ersten Gesundheitsversorgungsdaten abgerufen werden können, basierend auf der ersten Vielzahl von Assoziationen;
selektiv, basierend auf einem Ergebnis des Bestimmens, bewirken, dass eine zweite Assoziation zwischen der zweiten Datenquelle und den angeforderten ersten Gesundheitsversorgungsdaten erkannt und in der Datenbank gespeichert wird;
Abrufen der angeforderten ersten Gesundheitsversorgungsdaten von der zweiten Datenquelle; und
Übertragen der abgerufenen ersten Gesundheitsversorgungsdaten zum Empfang durch die Benutzervorrichtung (101).

## Revendications

1. Système (103) pouvant fonctionner pour procurer des données de soins de santé à un dispositif (101) utilisateur dans un réseau (100) de soins de santé, le réseau (100) de soins de santé comprenant une pluralité de sources (107) de données comprenant des données de soins de santé, le système comprenant au moins un processeur et au moins une mémoire ayant un code de programme d'ordinateur, la au moins une mémoire et le code de programme d'ordinateur étant configurés pour, avec le au moins un processeur, faire que le système (103) effectue au moins les stades de :
maintien, dans une base (105) de données, d'une première pluralité d'associations entre une première pluralité des sources de données et des données de soins de santé, qui sont mises en mémoire ;
réception, provenant du dispositif (101) utilisateur, d'une demande d'une première donnée de soins de santé, dans lequel la première donnée de soins de santé demandée est mise en mémoire dans une deuxième des sources (107) de données, la deuxième source de données étant différente de la première pluralité de sources de données ;
détermination du point de savoir si la première donnée de soins de santé demandée peut être retrouvée sur la base de la première pluralité d'associations ;
sélectivement, sur la base d'un résultat de la détermination, faire en sorte qu'une deuxième association entre la deuxième source de données et la première donnée de soins de santé demandée soit détectée et mise en mémoire dans la base (105) de données ;
recherche de la première donnée de soins de santé demandée dans la deuxième source de données ; et
transmission de la première source de soins de données recherchée pour réception par le dispositif (101) utilisateur.

2. Système (103) suivant la revendication 1, dans lequel la au moins une mémoire et le code de programme d'ordinateur sont configurés pour, avec le au moins un processeur, faire que le système (103) effectue le stade de :
faire en sorte que la deuxième association soit détectée si une association correspondant à la première donnée de soins de santé demandée n'est pas déterminée sur la base de la première pluralité d'associations.

3. Système (103) suivant la revendication 1 ou 2, dans lequel la au moins une mémoire et le code de programme d'ordinateur sont configurés pour, avec le moins un processeur, faire en sorte que le système (103) effectue les stades de :
réception en provenance du dispositif (101) utilisateur, d'une demande d'une deuxième donnée de soins de santé, dans lequel la deuxième donnée de soins de santé est mise en mémoire dans une source de données de la première pluralité de sources de données ;
détermination que la deuxième donnée de soins de santé peut être recherchée sur la base d'une association de la première pluralité d'associations ;
recherche de la deuxième donnée de santé demandée sur la base de l'association déterminée ; et
transmission de la deuxième donnée de soins de santé recherchée pour réception par le dispositif (101) utilisateur.

4. Système (103) suivant l'une quelconque des revendications 1 à 3, dans lequel la au moins une mémoire et le code de programme d'ordinateur sont configurés pour, avec le au moins un processeur, faire en sorte que le système (103) effectue le stade de :
utilisation d'une application (303) pour détecter celle des sources (107) de données qui comprend la donnée de soins de santé demandée, en détectant ainsi la deuxième association.

5. Système suivant la revendication 4, dans lequel l'application (303) comprend une fonctionnalité d'exploration web.

6. Système (103) suivant l'une quelconque des revendications 1 à 5, dans lequel la au moins une mémoire et le code de programme d'ordinateur sont configurés pour, avec le au moins un processeur, faire en sorte que le système (103) effectue le stade de :
réception, en provenance du dispositif (101) utilisateur d'une autre demande de données de soins de santé, l'autre donnée de soins de santé demandée étant mise en mémoire dans la deuxième source de données ;
recherche de l'autre donnée de soins de santé demandée sur la base de la deuxième association ; et
transmission de l'autre donnée de soins de santé demandée pour réception par le dispositif (101) utilisateur.

7. Système (103) suivant l'une quelconque des revendications 1 à 6, dans lequel la au moins une mémoire et le code de programme d'ordinateur sont configurés pour, avec le au moins un processeur, faire en sorte que le système (103) effectue le stade de :
retrait d'une association de la première pluralité d'associations, en mettant fin ainsi à l'accès de la donnée de soins de santé correspondante par l'intermédiaire du réseau (100) de soins de santé.

8. Système (103) suivant l'une quelconque des revendications 1 à 7, dans lequel la au moins une mémoire et le code de programme d'ordinateur sont configurés pour, avec le au moins un processeur, faire en sorte que le système (103) effectue le stade de :
en réaction à la réception d'une donnée indicatrice d'un changement entre la deuxième source de données et la donnée de soins de santé, qui y est mise en mémoire, mise à jour de la deuxième association, en continuant ainsi à faciliter l'accès à la donnée de soins de santé mise en mémoire dans la deuxième source de données par l'intermédiaire du réseau (100) de soins de santé.

9. Système (103) suivant l'une quelconque des revendications 1 à 8, dans lequel la au moins une mémoire et le code de programme d'ordinateur sont configurés pour, avec le au moins un processeur, faire en sorte que le système (103) effectue le stade de :
création d'une carte virtuelle de la première pluralité de sources de données et de la deuxième source de donnée sur la base de la base de la première pluralité d'associations et de la deuxième association, en créant ainsi un modèle (500) de patient.

10. Système (103) suivant la revendication 9, dans lequel la au moins une mémoire et le code de programme d'ordinateur sont configurés pour, avec le au moins un processeur, faire en sorte que le système (103) effectue les stades de :
en réaction à la réception de données associées à un patient, faire en sorte que le dispositif (101) utilisateur acquiert une donnée de soins de santé sur la base d'un paramètre d'état et utiliser le modèle (500) de patient, le paramètre d'état étant indicateur de l'un des stades (501 à 517) différents associés à l'analyse de la donnée reçue ; et
faire en sorte qu'au moins une partie de la donnée de soins de santé acquise soit affichée sur le dispositif (101) utilisateur.

11. Système (103) suivant la revendication 10, dans lequel la au moins une mémoire et le code de programme d'ordinateur sont configurés pour, avec le au moins un processeur, faire en sorte que le système (103) effectue les stades de :
contrôle d'actions antérieures d'un utilisateur lors de l'analyse de la donnée de soins de santé associée à au moins certains des stades (501 à 517) différents ; et
commander l'affichage d'au moins une partie de la donnée de soins de santé acquise sur le dispositif (101) utilisateur, sur la base d'une combinaison du paramètre d'état et du contrôle.

12. Système (103) suivant les revendications 10 ou 11, dans lequel la au moins une mémoire et le code de programme d'ordinateur sont configurés pour, avec le au moins un processeur, faire en sorte que le système (103) effectue les stades de :
en réaction à une entrée d'utilisateur, faire en sorte que le dispositif (101) utilisateur acquiert une autre donnée de soins de santé sur la base d'une combinaison de l'entrée et du paramètre d'état et utiliser le modèle (500) de patient ; et
faire en sorte que le dispositif (101) utilisateur présente au moins une partie de l'autre donnée de soins de santé acquise.

13. Système (103) suivant l'une quelconque des revendications 10 à 12, dans lequel la au moins une mémoire et le code de programme d'ordinateur sont configurés pour, avec le au moins un processeur, faire en sorte que le système (103) effectue les stades de :
faire en sorte que le dispositif (101) utilisateur traite la donnée de santé acquise sur la base d'un paramètre déterminé à l'avance ; et
faire en sorte qu'au moins une partie de la donnée de soins de santé traitée soit affichée sur le dispositif (101) utilisateur.

14. Système (103) suivant la revendication 13, dans lequel le paramètre déterminé à l'avance comprend :
une donnée associée au patient ;
une donnée associée à un autre patient ;
une donnée indicatrice d'un utilisateur de soins de santé ;
une donnée indicatrice d'un état médical ;
une donnée indicatrice d'une ou plusieurs directives ;
une donnée indicatrice d'une ou plusieurs règles légales ;
une donnée indicatrice d'un paramètre de personnalisation ; et/ou
une donnée indicatrice d'un réglage d'utilisateur.

15. Système (103) suivant l'une quelconque des revendications 10 à 14, dans lequel la au moins une mémoire et le code de programme d'ordinateur sont configurés pour, avec le au moins un processeur, faire en sorte que le système effectue les stades de :
maintien d'un paramètre de contexte indicateur d'un stade clinique associé à une analyse de la donnée reçue ;
filtrage de la donnée de soins de santé acquise sur la base du paramètre de contexte ; et
faire en sorte qu'au moins une partie de la donnée de soins de santé filtrée soit affichée sur le dispositif (101) utilisateur.

16. Système (103) suivant la revendication 15, dans lequel la au moins une mémoire et le code de programme d'ordinateur sont configurés pour, avec le au moins un processeur, faire en sorte que le système (103) effectue le stade de :
commande de l'affichage d'au moins une partie de la donnée de soins de santé filtrée sur le dispositif (101) utilisateur sur la base d'une combinaison du paramètre d'état et du paramètre de contexte.

17. Système (103) suivant l'une quelconque des revendications 10 à 16, dans lequel la donnée de soins de santé est acquise sur la base de :
une donnée indicatrice du patient ;
une donnée indicatrice d'un utilisateur de soins de santé ;
une donnée indicatrice d'un état médical ;
une donnée indicatrice d'une ou plusieurs directives ;
une donnée indicatrice d'une ou plusieurs règles légales ;
une donnée indicatrice d'un paramètre de personnalisation ; et/ou
une donnée indicatrice d'un réglage d'utilisateur.

18. Système (103) suivant l'une quelconque des revendications 10 à 17, dans lequel la donnée de soins de santé acquise comprend une donnée indicatrice :
d'une donnée de santé associée au patient ;
d'une donnée de soins santé associée à un autre patient ; et/ou d'un savoir médical d'expert.

19. Système (103) suivant l'une quelconque des revendications 1 à 18, dans lequel les sources (107) de données comprennent :
une source de donnée d'Electronic Medical Records (EMR) ;
une source de donnée de Picture Archiving Communication Systems (PACS) ;
une source de données d'Emergency Care Summary (ECS) ;
une source de données de Key Information Summary (KIS) ;
une source de données d'Electronic Healthcare Record (EHR) ;
une source de données d'Electronic Patient Record (EPR) et/ou
une source de données de Personal Health Record (PHR).

20. Système (103) suivant l'une quelconque des revendications 1 à 19, dans lequel le système (103) est configuré pour communiquer avec une première des sources (107) de données en utilisant une interface (305) de programmation d'application en accord avec une norme Fast Healthcare Interoperability Resources (FHIR).

21. Procédé pour procurer une donnée de soins de santé à un dispositif (101) utilisateur dans un réseau (100) de soins de santé, le réseau (100) de soins de santé comprenant une pluralité de sources (107) de données, comprenant des données de soins de santé, le procédé comprenant :
maintenir, dans une base (105) de données, une première pluralité d'associations entre une première pluralité des sources de données et des données de soins de santé, qui y sont mémorisées ;
recevoir du dispositif (101) utilisateur une demande d'une première donnée de soins de santé, dans lequel la première donnée de soins de santé demandée est mise en mémoire dans une deuxième des sources (107) de données, la deuxième source de données étant différente de la première pluralité de sources de données ;
déterminer si la première donnée de soins de santé demandée peut être recherchée sur la base de la première pluralité d'associations ;
sélectivement, sur la base du résultat de la détermination, faire en sorte qu'une deuxième association entre la deuxième source de données et la première donnée de soins de santé demandée soit détectée et mise en mémoire dans la base (105) de données ;
rechercher la première donnée de soins de santé demandée dans la deuxième source de données ; et
transmettre la première donnée de soins de santé recherchée pour réception par le dispositif (101) utilisateur.

22. Programme d'ordinateur comprenant un ensemble d'instructions, qui, lorsqu'elles sont exécutées par un dispositif informatique, font que le dispositif informatique effectue un procédé procurant des données de soins de santé à un dispositif (101) utilisateur dans un réseau (100) de soins de santé, le réseau de soins de santé comprenant une pluralité de sources de données comprenant des données de soins de santé, le procédé comprenant, au dispositif informatique :
maintenir dans une base (105) de données, une première pluralité d'associations entre une première pluralité des sources de données et des données de soins de santé, qui y sont mémorisées ;
recevoir du dispositif (101) utilisateur une demande d'une première donnée de soins de santé, dans lequel la première donnée de soins de santé demandée est mise en mémoire dans une deuxième des sources (107) de données, la deuxième source de données étant différente de la première pluralité de sources de données ;
déterminer si la première donnée de soins de santé demandée peut être recherchée sur la base de la première pluralité d'associations ;
sélectivement, sur la base du résultat de la détermination, faire en sorte qu'une deuxième association entre la deuxième source de données et la première donnée de soins de santé demandée soit détectée et mise en mémoire dans la base de données ;
rechercher la première donnée de soins de santé demandée dans la deuxième source de données ; et
transmettre la première donnée de soins de santé recherchée pour réception par le dispositif (101) utilisateur.
